# EUROPEAN PATENT APPLICATION

(11) **EP 4 846 944 A1**
(43) Date of publication of application: **02.12.2026**
(21) Application number: 26181692.0
(22) Date of filing: 28.05.2026
(51) Int. Cl.: A61B 8/06, A61B 8/00, A61B 8/08

(54) **ULTRASOUND DIAGNOSTIC APPARATUS, MEDICAL INFORMATION PROCESSING APPARATUS, AND PROGRAM**

(30) Priority: 28.05.2025 JP 2025089313
(71) Applicant: Canon Kabushiki Kaisha, Tokyo 146-8501 (JP)
(72) Inventor: IKEDA, Hayato, Tokyo, 146-8501 (JP); KAKEE, Akihiro, Tokyo, 146-8501 (JP); IWASAKI, Ryosuke, Tokyo, 146-8501 (JP)
(74) Representative: Marks & Clerk LLP

(57) **Abstract**

An ultrasound diagnostic apparatus (110) according to the embodiments includes an ultrasound probe (105) and a processing circuit (150). The processing circuit (150) is configured to generate first ultrasound data (10) representing a fluid inside a body of a subject based on a received signal of an ultrasound wave received by the ultrasound probe (105), generate second ultrasound data (3) by performing processing of locally extracting or enhancing the fluid represented by the first ultrasound data (10), and display, on a display (135), a combination of a first ultrasound image based on the first ultrasound data (10) and a second ultrasound image based on the second ultrasound data (3), or a combination of a plurality of the second ultrasound images based on a plurality of pieces of the second ultrasound data (3).

## Description

### FIELD

Embodiments disclosed herein relate generally to an ultrasound diagnostic apparatus, a medical information processing apparatus, a method and a program.

### BACKGROUND

Conventionally, techniques for improving the image quality of ultrasound images containing fine flow channels and fluids (for example, blood flow, contrast agents) have been known. For example, Patent Document 1 (Japanese Unexamined Patent Application Publication No. 2001-178720) discloses that an image representing the boundary of a flow channel (for example, the shape of a blood vessel) is generated by removing clutter contained in received signals of continuous ultrasound waves within a predetermined time period and averaging the fluid signals after clutter removal over a predetermined time period. Furthermore, Patent Document 2 (Japanese Translation of PCT International Application Publication No. 2019-526350) discloses that a flow channel of fluid is displayed in high image quality by performing super-resolution processing that improves the resolution (pixel density) of an ultrasound image and sharpens the peaks of speckle patterns in the ultrasound image, and superimposing a plurality of peak-sharpened speckle patterns within a predetermined time period.

In this way, in a case where processing for improving the image quality of an ultrasound image is performed, the fluid represented by the original ultrasound image is locally extracted or enhanced, so that contrast or sharpness is improved but sensitivity is reduced, and noise is reduced but responsiveness is degraded, which presents a trade-off problem.

### SUMMARY OF INVENTION

An ultrasound diagnostic apparatus according to an aspect of the present invention comprises: an ultrasound probe; and a processing circuit. The processing circuit is configured to
generate first ultrasound data representing a fluid inside a body of a subject based on a received signal of an ultrasound wave received by the ultrasound probe,
generate second ultrasound data by performing processing of locally extracting or enhancing the fluid represented by the first ultrasound data, and
display a combination of a first ultrasound image based on the first ultrasound data and a second ultrasound image based on the second ultrasound data, or a combination of a plurality of the second ultrasound images based on a plurality of pieces of the second ultrasound data on a display.

The processing circuit may be configured to
generate the second ultrasound data representing a single image in which the fluid is locally extracted or enhanced, from the first ultrasound data and representing a plurality of images within a predetermined time period, and
display the combination of the first ultrasound image based on the first ultrasound data and the second ultrasound image based on the second ultrasound data on the display.

The processing circuit may be configured to
superimpose the first ultrasound image and the second ultrasound image within a predetermined display range of the display,
display a portion of the first ultrasound image corresponding to one region of the display range in a cropped state in the one region, and
display a portion of the second ultrasound image corresponding to an other region of the display range in a cropped state in the other region.

The processing circuit may be configured to display the first ultrasound image and the second ultrasound image side by side on the display.

The processing circuit may be configured to display the first ultrasound image and the second ultrasound image on the display in a switched manner.

The processing circuit may be configured to
generate two pieces of the second ultrasound data, each representing a single image in which the fluid is locally extracted or enhanced, from the first ultrasound data representing a first number of images and the first ultrasound data representing a second number of images greater than the first number, and
display a combination of two second ultrasound images based on the two pieces of the second ultrasound data on the display.

The processing circuit may be configured to
superimpose the two second ultrasound images within a predetermined display range of the display,
display a portion of one of the second ultrasound images corresponding to one region of the display range in a cropped state in the one region, and
display a portion of an other of the second ultrasound images corresponding to an other region of the display range in a cropped state in the other region.

The processing circuit may be configured to display the two second ultrasound images side by side on the display.

The processing circuit may be configured to display the two second ultrasound images on the display in a switched manner.

A medical information processing apparatus according to an aspect of the present invention comprises a processing circuit configured to
generate first ultrasound data representing a fluid inside a body of a subject based on a received signal of an ultrasound wave received by an ultrasound probe,
generate second ultrasound data by performing processing of locally extracting or enhancing the fluid represented by the first ultrasound data, and
display a combination of a first ultrasound image based on the first ultrasound data and a second ultrasound image based on the second ultrasound data, or a combination of a plurality of the second ultrasound images based on a plurality of pieces of the second ultrasound data on a display.

A program according to an aspect of the present invention causes a computer to execute:
generating first ultrasound data representing a fluid inside a body of a subject based on a received signal of an ultrasound wave received by an ultrasound probe;
generating second ultrasound data by performing processing of locally extracting or enhancing the fluid represented by the first ultrasound data; and
displaying, on a display, a combination of a first ultrasound image based on the first ultrasound data and a second ultrasound image based on the second ultrasound data, or a combination of a plurality of the second ultrasound images based on a plurality of pieces of the second ultrasound data.

### BRIEF DESCRIPTION OF THE DRAWINGS

FIG. 1 is a diagram illustrating an example of a configuration of an ultrasound diagnostic apparatus according to a first embodiment;
FIG. 2 is a flowchart illustrating a flow of processing performed by the ultrasound diagnostic apparatus illustrated in FIG. 1;
FIG. 3 is a diagram illustrating the processing according to the first embodiment;
FIG. 4 is a diagram illustrating the processing according to the first embodiment;
FIG. 5 is a diagram illustrating the processing according to the first embodiment;
FIG. 6 is a flowchart illustrating a flow of processing performed by an ultrasound diagnostic apparatus according to a second embodiment;
FIG. 7 is a diagram illustrating the processing according to the second embodiment;
FIG. 8 is a diagram illustrating processing according to a third embodiment; and
FIG. 9 is a diagram illustrating processing according to another embodiment.

### DETAILED DESCRIPTION

An ultrasound diagnostic apparatus according to the embodiments includes an ultrasound probe and a processing circuit. The processing circuit is configured to generate first ultrasound data representing a fluid inside a body of a subject based on a received signal of an ultrasound wave received by the ultrasound probe, generate second ultrasound data by performing processing of locally extracting or enhancing the fluid represented by the first ultrasound data, and display, on a display, a combination of a first ultrasound image based on the first ultrasound data and a second ultrasound image based on the second ultrasound data, or a combination of a plurality of the second ultrasound images based on a plurality of pieces of the second ultrasound data.

### First Embodiment

Hereinafter, embodiments of an ultrasound diagnostic apparatus, a medical information processing apparatus, and a computer program will be described with reference to the drawings.

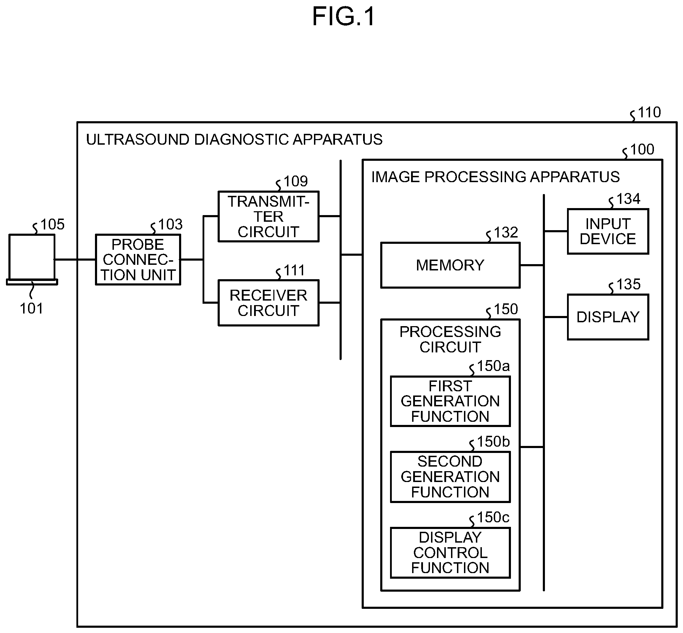

First, the configuration of an ultrasound diagnostic apparatus 110 will be described with reference to FIG. 1. The ultrasound diagnostic apparatus 110 generates ultrasound data based on a received signal (reflected wave signal) received from the ultrasound probe 105. The ultrasound diagnostic apparatus 110 illustrated in FIG. 1 can generate ultrasound data representing a two-dimensional ultrasound image based on a received signal received from a one-dimensional ultrasound probe 105 (an ultrasound probe in which transducer elements are arranged in one dimension), and can generate ultrasound data representing a three-dimensional ultrasound image based on a received signal received from a two-dimensional ultrasound probe 105 (an ultrasound probe in which transducer elements are arranged in two dimensions).

The ultrasound probe 105 transmits ultrasound waves toward a subject and receives the ultrasound waves reflected by the subject. The ultrasound probe 105 is, for example, an electronic scanning probe, and has a plurality of transducer elements 101 arranged in one dimension or two dimensions at a distal end thereof. Each of the transducer elements 101 is a piezoelectric element (electromechanical transduction element) that performs interconversion between an electrical signal (voltage pulse signal) and an ultrasound wave (acoustic wave). The ultrasound probe 105 transmits ultrasound waves toward the subject from the transducer elements 101, and receives the reflected ultrasound waves from the subject by the transducer elements 101. The reflected acoustic waves reflect differences in acoustic impedance within the subject. When a transmitted ultrasound pulse is reflected by a surface of moving blood flow, a heart wall, or the like, the reflected ultrasound waves undergo a frequency shift due to the Doppler effect depending on a velocity signal component of a moving object with respect to an ultrasound transmission direction.

The ultrasound diagnostic apparatus 110 includes a probe connection unit 103, a transmitter circuit 109, a receiver circuit 111, and an image processing apparatus 100.

The probe connection unit 103 connects to the ultrasound probe 105 and transmits and receives ultrasound signals (electrical signals) to and from the ultrasound probe 105. A connection means for connecting the ultrasound probe 105 by the probe connection unit 103 may be either wired or wireless. In a wired configuration, the probe connection unit 103 includes a connector part (receptacle) connecting to a connector (plug) of the ultrasound probe 105. In a wireless configuration, the probe connection unit 103 includes a communication unit that performs wireless communication with the ultrasound probe 105.

The transmitter circuit 109 is a transmission unit that outputs pulse signals (drive signals) to the transducer elements 101. Pulse signals are applied to the transducer elements 101 with respective time differences, and ultrasound waves having different delay times are transmitted from the transducer elements 101, to form a transmitted ultrasound beam. The direction and focus of the transmitted ultrasound beam can be controlled by selectively changing the transducer elements 101 to which pulse signals are applied (that is, the transducer elements 101 to be driven) and by changing delay times (application timings) of the pulse signals. An observation region inside the subject is scanned while the direction and focus of the transmitted ultrasound beam are sequentially changed. A transmitted ultrasound beam that is a plane wave (with a far focus) or a diverging wave (with a focal point located in a direction opposite to the ultrasound transmission direction with respect to the transducer elements 101) may be formed by changing delay times of the pulse signals. Alternatively, a transmitted ultrasound beam may be formed using a single transducer element or some of the transducer elements 101. The transmitter circuit 109 transmits pulse signals having a predetermined drive waveform to the transducer elements 101, to generate transmitted ultrasound waves having a predetermined transmission waveform at the transducer elements 101.

The receiver circuit 111 is a reception unit that inputs, as received signals, electrical signals output from the transducer elements 101 that have received reflected ultrasound waves. The received signals are input to processing circuit 150. In the present embodiment, both analog signals output from the transducer elements 101 and digital data obtained by sampling (digital conversion) the analog signals are referred to as received signals without particular distinction.

The image processing apparatus 100 is connected to the transmitter circuit 109 and the receiver circuit 111, and performs processing on signals received from the receiver circuit 111 and controls the transmitter circuit 109. The image processing apparatus 100 includes a processing circuit 150, a memory 132, an input device 134, and a display 135.

The memory 132 includes a random access memory (RAM), a semiconductor memory device such as a flash memory, a hard disk, an optical disc, and the like. The memory 132 is a memory that stores therein data such as image data for display generated by the processing circuit 150. The memory 132 may also store received signals (reflected wave signals) output from the receiver circuit 111. In addition, the memory 132 stores therein, as necessary, various types of data such as a control program for performing ultrasound transmission and reception, image processing, and display processing, diagnostic information (for example, a patient ID and a physician's findings), diagnostic protocols, and various body marks.

The input device 134 receives various instructions and information input from an operator. The input device 134 includes, for example, input interface devices such as a trackball, switch buttons, a mouse, a keyboard, a touch pad that performs input operations by touch on an operation surface, a touch monitor in which a display screen and a touch pad are integrated, a non-contact input circuit using an optical sensor, and a voice input circuit. The input interface device is connected to the processing circuit 150 described later, and convert input operations received from an operator into electrical signals and output the electrical signals to the processing circuit 150. In the present specification, the input interface device is not limited to devices including physical operation components such as a mouse and a keyboard. For example, an electrical signal processing circuit that receives electrical signals corresponding to input operations from an external input device provided separately from the apparatus and outputs the electrical signals to the processing circuit 150 is also included as an example of the input interface device.

The display 135 displays, under control of the processing circuit 150, a graphical user interface (GUI) for receiving input of imaging conditions, and various images. The display 135 includes a display interface device such as, for example, a liquid crystal display.

The processing circuit 150 controls the entire ultrasound diagnostic apparatus 110 by controlling each unit of the ultrasound diagnostic apparatus 110. For example, the processing circuit 150 executes a computer program stored in the memory 132, to implement a first generation function 150a, a second generation function 150b, and a display control function 150c. Here, the first generation function 150a is an example of a first generation unit. The second generation function 150b is an example of a second generation unit. The display control function 150c is an example of a display control unit. In FIG. 1, the processing circuit 150 is described as being implemented as a single unit; however, the processing circuit 150 may be implemented by a plurality of independent processors in combination. In addition, a specific function such as an application specific integrated circuit (ASIC) may be constituted by a dedicated independent circuit.

The term "processor" used in the above description means, for example, a circuit such as a central processing unit (CPU), a graphical processing unit (GPU), an application specific integrated circuit (ASIC), or a programmable logic device (for example, simple programmable logic device (SPLD), complex programmable logic device (CPLD), and field programmable gate array (FPGA)). The processor reads and executes computer programs stored in the memory 132 to execute the functions thereof.

The ultrasound diagnostic apparatus 110 configured as described above performs the following processing:
(Processing 1) first generation processing of generating first ultrasound data representing a fluid (blood, contrast agent, lymph fluid, and other fluids) inside a body of a subject based on a received signal of an ultrasound wave received by the ultrasound probe 105;
(Processing 2) second generation processing of generating second ultrasound data by performing processing of locally extracting or enhancing the fluid represented by the first ultrasound data; and
(Processing 3) display control processing of displaying, on the display 135, a combination of a first ultrasound image based on the first ultrasound data and a second ultrasound image based on the second ultrasound data.

Processing 2 corresponds to processing for improving image quality of the first ultrasound data (for example, power Doppler data) representing a fluid (for example, blood flow) inside the body of the subject, the first ultrasound data being obtained by Processing 1. In Processing 2, since the fluid represented by the first ultrasound data is locally extracted or enhanced, contrast or sharpness is improved but sensitivity may be reduced, and noise is reduced but responsiveness may be lowered. In Processing 3, the combination of the first ultrasound image based on the first ultrasound data and the second ultrasound image based on the second ultrasound data is displayed on the display 135. Accordingly, the second ultrasound image, in which contrast or sharpness is improved and noise is reduced, is displayed on the display 135 together with the first ultrasound image having higher sensitivity and responsiveness than the second ultrasound image, and the trade-off problem caused by Processing 2 is thus reduced.

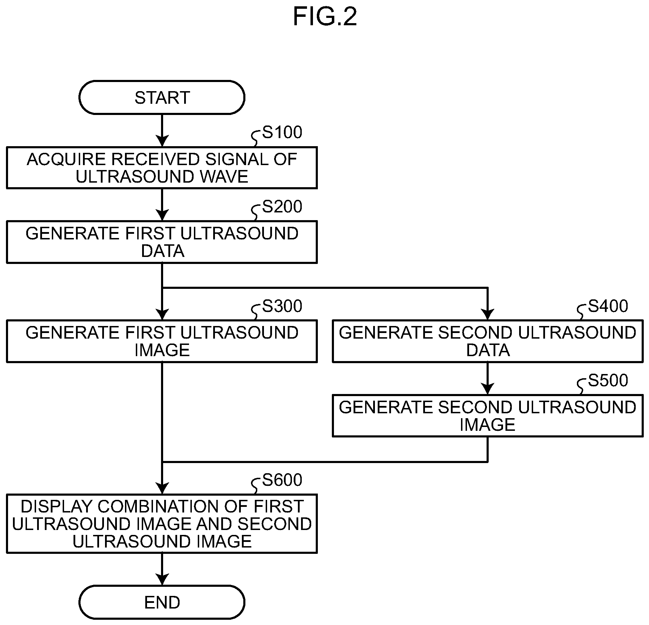

Hereinafter, the operation of the ultrasound diagnostic apparatus 110 will be described with reference to FIGS. 2 to 5. FIG. 2 is a flowchart illustrating a flow of processing performed by the ultrasound diagnostic apparatus 110.

The first generation function 150a controls the transmitter circuit 109 and the receiver circuit 111 to cause the ultrasound probe 105 to perform an ultrasound scan and to acquire a received signal of an ultrasound wave (for example, CH data) (Step S100). Specifically, the first generation function 150a initiates control to collect, at a predetermined frame rate, a plurality of pieces of frame data (a plurality of pieces of frame data within a predetermined time) continuous in a time direction and obtained by execution of the ultrasound scan. The frame data includes amplitude values (signal values) of signals corresponding to respective pixels constituting a single image (frame).

The first generation function 150a performs delay-and-sum and quadrature detection processing on the collected received signals. The delay-and-sum processing is processing in which received signals from the transducer elements 101 are added together while varying delay times and weights for each transducer element 101, and is also referred to as delay-and-sum (DAS) beamforming. The quadrature detection processing is processing for converting a received signal into an in-phase signal and a quadrature signal in a baseband to acquire measurement data representing IQ data or an absolute value of the IQ data. In addition, adaptive beamforming, model-based processing, machine learning-based processing, and the like may be performed on the received signals.

In a case where a plurality of pieces of frame data are collected, the first generation function 150a estimates a displacement amount of tissue due to body motion of the subject or the like between the pieces of frame data, and corrects each piece of frame data based on the estimated result. Specifically, the first generation function 150a calculates a displacement amount of tissue due to body motion or the like between frames from the pieces of frame data, and corrects the frame data based on the calculated displacement amount.

The first generation function 150a generates blood flow data (power Doppler data) obtained by extracting information derived from blood flow from the measurement data described above. For example, the processing circuit 150 applies a moving target indicator (MTI) filter to each collected piece of frame data (Step S203). As a result, information (clutter) derived from stationary tissue or slow-moving tissue between frames is reduced, and information derived from blood flow (blood flow signal components) is extracted. As the MTI filter, a filter with fixed filter information, such as a Butterworth-type infinite impulse response (IIR) filter or a polynomial regression filter, may be used. The MTI filter may be an adaptive filter that varies its coefficients in accordance with an input signal using eigenvalue decomposition or singular value decomposition.

The first generation function 150a may also decompose the frame data into a plurality of bases using eigenvalue decomposition or singular value decomposition, extract a specific basis, thereby removing information derived from tissue and extracting information derived from blood flow. The first generation function 150a may also determine a velocity vector at each coordinate within the received signal data by using techniques such as a vector Doppler method, a speckle tracking method, and a vector flow mapping method, and may determine a blood flow vector representing a magnitude and a direction of blood flow. In addition to the methods exemplified herein, any method capable of extracting blood flow-derived information included in the frame data (measurement data) or removing tissue-derived information may be employed. The first generation function 150a can also generate B-mode data representing signal intensity at each point within an observation region as brightness by performing envelope detection processing, logarithmic compression processing, or the like on the measurement data.

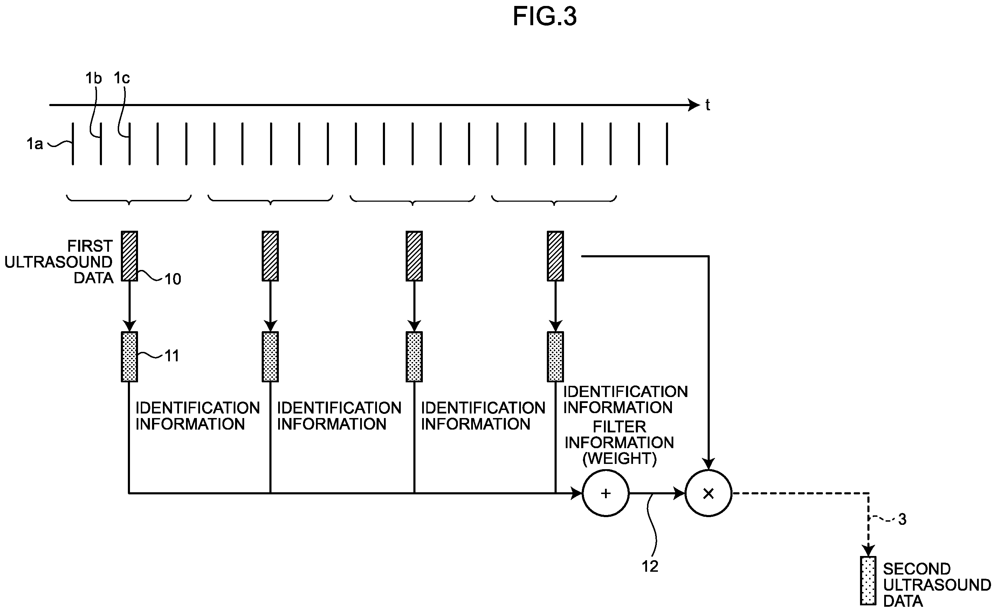

For example, as illustrated in FIG. 3, the first generation function 150a sequentially generates a plurality of pieces of frame data 1a, 1b, and 1c that are continuous in a time direction based on received signals of ultrasound waves received by the ultrasound probe 105. The frame data includes amplitude values (power signal values) of blood flow signals (fluid signals) corresponding to respective pixels constituting a single image (frame). The first generation function 150a also generates first ultrasound data 10 (data representing a single image) by performing addition/time averaging (persistence) for each predetermined number of frames (five frames in the example illustrated in FIG. 3) (Step S200). The predetermined number of frames (number of packets) may be, for example, approximately several frames to one hundred frames. As described above, the first generation function 150a generates the first ultrasound data 10 representing the fluid inside the body of the subject based on the received signals of the ultrasound waves received by the ultrasound probe 105. The first generation function 150a generates a first ultrasound image 20 based on the first ultrasound data (Step S300).

The first ultrasound data 10 has a higher signal-to-noise ratio (SNR) than each of the pieces of the frame data 1a, 1b, and 1c does. The first ultrasound data 10 is, for example, blood flow data (power Doppler data) in which information derived from blood flow is enhanced. The first ultrasound data 10 also corresponds to a single image representing a boundary of a flow channel (for example, a shape of a blood vessel), in which signals of a fluid after clutter removal are averaged over a predetermined time period. The first ultrasound data 10 includes signal values (amplitude values) representing a speckle pattern generated by constructive interference and destructive interference between ultrasound waves reflected from the fluid, as well as signal values representing other noise (artifacts and the like) that occur instantaneously or intermittently along a time axis.

The second generation function 150b generates second ultrasound data 3 by performing processing of locally extracting or enhancing a fluid represented by the first ultrasound data 10 (Step S400). This processing serves to improve image quality of an image (first ultrasound image 20) represented by the first ultrasound data 10 generated at Step S200. In the present embodiment, the second generation function 150b generates the second ultrasound data 3 representing a single image in which the fluid is locally extracted or enhanced, from the first ultrasound data 10 generated by the first generation function 150a and representing a plurality of images (frames) within a predetermined time period. That is, the second generation function 150b generates the second ultrasound data 3 representing the single image based on the first ultrasound data 10 representing the images continuous in a time direction.

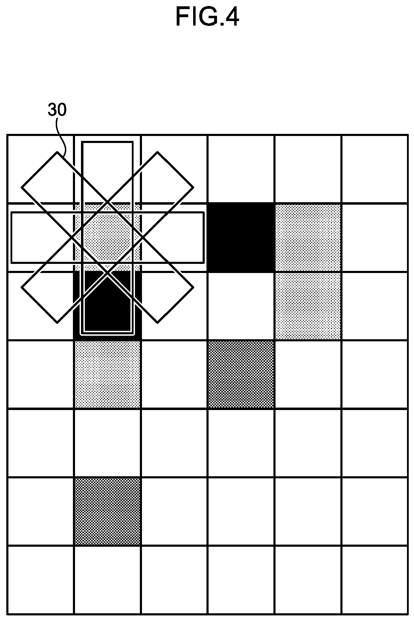

The second generation function 150b sequentially generates filter information 12 representing local peak positions of the speckle pattern by analyzing the first ultrasound data 10. The second generation function 150b performs processing of applying the filter information 12 to the first ultrasound data 10. Specifically, as illustrated in FIG. 3, for each position of each frame (image) represented by each of a plurality of pieces (for four frames) of the first ultrasound data 10, the second generation function 150b acquires information (identification information 11) for identifying a magnitude relationship between a signal value corresponding the position and signal values corresponding to a periphery of the position. Specifically, as illustrated in FIG. 4, the second generation function 150b arranges a kernel 30 at a position of interest in a frame (image) represented by the first ultrasound data 10, and compares a magnitude relationship between a signal value corresponding to the position of interest and signal values corresponding to positions of the image within a range where the kernel 30 is arranged (a periphery of the position of interest). The second generation function 150b acquires, for example, for each position of a frame, information for identifying whether or not a signal value corresponding to the position is greater than signal values corresponding to a periphery of the position, as the identification information. That is, the identification information 11 can be referred to as information for identifying a position where a signal value is relatively large (local peak position) within a local range of the frame.

Specifically, as illustrated in FIG. 4, in a case where the kernel 30 based on a ratio of 3 vertical pixels by 1 horizontal pixel is used, the second generation function 150b arranges a center (second square) of the kernel 30 at a position of interest, and identifies whether or not a signal value corresponding to the position of interest is greater than signal values corresponding to positions at both ends (first square and third square) of the kernel 30. For example, in a case where the signal value corresponding to the position of interest is greater than the signal values corresponding to the periphery of the position, the second generation function 150b represents the identification information by "1", and in a case where the signal value corresponding to the position of interest is smaller than the signal values corresponding to the periphery of the position, the second generation function 150b represents the identification information by "0". The second generation function 150b extracts the identification information for each position of the image (frame) represented by the first ultrasound data 10, to extract the identification information 11 of the entire image (frame) represented by the first ultrasound data 10. That is, through this extraction processing, the first ultrasound data 10 is converted into a binary image in which each pixel is represented by "0" or "1".

In FIG. 4, the example is illustrated in which the identification information 11 is extracted by arranging the kernel 30 in four directions of vertical, horizontal, upper-right diagonal, and upper-left diagonal directions for each position of the frame represented by the first ultrasound data, but the shape, size, and arrangement of the kernel are not limited thereto. In FIG. 4, the example is also illustrated in which the object (fluid) is extracted in units of pixels, but the identification information may be extracted for each position of the frame in units of groups, each having a plurality of pixels. In a case where the identification information is extracted in units of groups, each having a plurality of pixels, an average value or an integrated value of signal values of respective positions constituting a group may be used as a signal value corresponding to each position of the frame.

Furthermore, the second generation function 150b generates the filter information 12 based on the identification information 11 corresponding to each of the images (frames) represented by the first ultrasound data 10. The filter information 12 is information that statistically represents local peak positions of the fluid in individual frames within a time period corresponding to a plurality of frames. The local peak positions of the fluid are positions where a signal value is relatively large within a local range of the frame. For example, the second generation function 150b generates the filter information 12 using the identification information 11 for four frames. The filter information 12 is obtained according to values represented by pieces of the identification information 11 at positions (coordinates (x, y)) corresponding to each other between frames. For example, in a case where all the values are "1" for all the four frames at positions corresponding to each other in the pieces of the identification information 11, the filter information for the positions is "1". In a case where "1" occurs three times and "0" occurs one time among the four frames at the positions corresponding to each other in the pieces of the identification information 11, the filter information for the positions is "0.75". In addition, in a case where "1" occurs two times and "0" occurs two times among the four frames at the positions corresponding to each other in the pieces of the identification information 11, the filter information for the positions is "0.5". In a case where "1" occurs one time and "0" occurs three times among the four frames at the positions corresponding to each other in the pieces of the identification information 11, the filter information for the positions is "0.25". In a case where "1" occurs zero times and "0" occurs four times among the four frames at the positions corresponding to each other in the pieces of the identification information 11, the filter information for the positions is "0".

As described above, in the pieces of the identification information 11 for a plurality of frames, as the number of times that the value is "1" at the positions corresponding to each other is larger, a larger signal value as compared with the periphery is continuously obtained at the positions, and a probability that the fluid exists is higher. Therefore, a large value (for example, "1", "0.75", or "0.5") is set for the filter information 12 corresponding to the positions. In contrast, in the pieces of the identification information 11 for a plurality of frames, as the number of times that the value is "0" at the positions corresponding to each other is larger, a probability that the fluid exists is lower at the positions. Therefore, a small value (for example, "0.25" or "0") is set for the filter information 12 corresponding to the positions.

The second generation function 150b generates the second ultrasound data 3 based on the filter information 12 and at least one piece of the first ultrasound data 10 of a plurality of pieces of first ultrasound data 21 (a plurality of pieces of frame data). For example, the second generation function 150b applies the filter information 12 to the latest piece of the first ultrasound data 10. That is, the second ultrasound data 3 is generated by multiplying a signal value (amplitude value) representing each position of a frame represented by the latest piece of the first ultrasound data 10 by a weighting factor set at each position of the filter information 12. That is, the second ultrasound data 3 is data obtained by converting the first ultrasound data 10 by using the filter information 12. The second generation function 150b generates a second ultrasound image 21 based on the second ultrasound data 3 (Step S500). In the second ultrasound image 21, the fluid represented by the first ultrasound data 10 is locally extracted or enhanced, so that contrast or sharpness is improved as compared with the first ultrasound image 20 but sensitivity may be reduced, and noise is reduced as compared with the first ultrasound image 20 but responsiveness may be lowered.

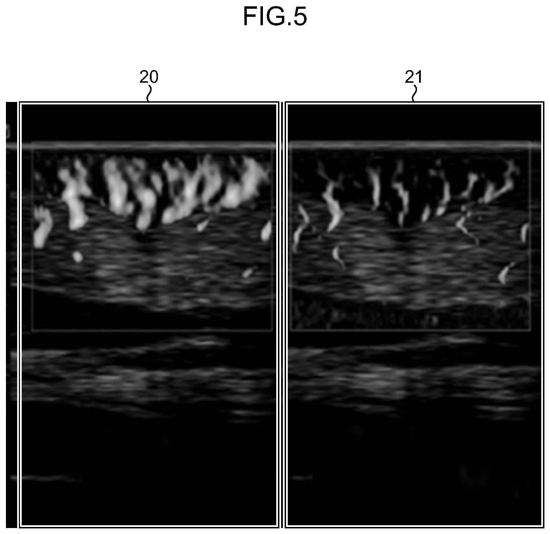

The display control function 150c displays a combination of the first ultrasound image 20 and the second ultrasound image 21 in respective predetermined display regions of the display 135 (Step S600). That is, a plurality of different images for the same part of the subject are displayed on the display 135. As illustrated in FIG. 5, the display control function 150c displays the first ultrasound image 20 and the second ultrasound image 21 side by side. The display control function 150c may switch and display the first ultrasound image 20 and the second ultrasound image 21 in a predetermined display region of the display 135.

As described above, according to the first embodiment, the second ultrasound image 21 in which contrast or sharpness is improved and noise is reduced is displayed on the display 135 together with the first ultrasound image 20 having higher sensitivity or responsiveness as compared with the second ultrasound image 21. Therefore, it is possible to reduce a problem of trade-off caused by processing for improving image quality of an ultrasound image.

In the present embodiment, the example in a case where the second ultrasound data 3 is generated by applying the filter information 12 to the latest piece of the first ultrasound data 10 has been described, but the second generation function 150b may generate the second ultrasound data 3 by applying the filter information 12 to at least one piece of the first ultrasound data 10 among the pieces of the first ultrasound data 21 (the pieces of the frame data). The second generation function 150b may also generate, as the second ultrasound data 3, data obtained by synthesizing the latest piece of the first ultrasound data 10 with data after applying the filter information 12 to at least one piece of the first ultrasound data 10 among the pieces of the first ultrasound data 21. In addition, the second generation function 150b may generate the second ultrasound data 3 by applying the filter information 12 to data obtained by performing addition or time averaging (persistence) on the pieces of the first ultrasound data 21 (the pieces of the frame data).

### Second Embodiment

In the first embodiment, the example in a case where the combination of the first ultrasound image 20 and the second ultrasound image 21 for the same part of the subject is displayed on the display 135 has been described. In the second embodiment, an example in a case where a combination of a plurality of pieces of the second ultrasound images 21 for the same part of the subject is displayed on the display 135 will be described.

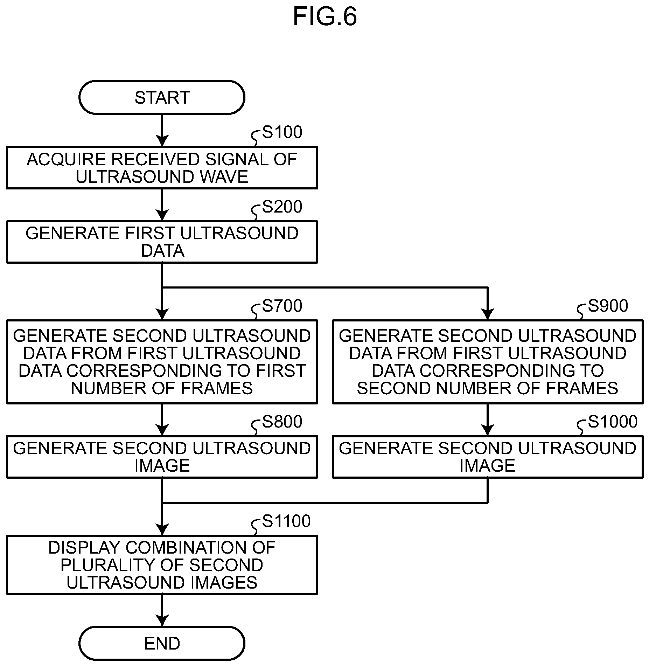

FIG. 6 is a flowchart illustrating a flow of processing performed by the ultrasound diagnostic apparatus 110. As for processing equivalent to the processing described in the first embodiment, the description thereof will not be repeated as appropriate.

When the first ultrasound data 10 is generated by the first generation function 150a (Step S200), the second generation function 150b generates second ultrasound data 3 from the first ultrasound data 10 representing the first number of images (Step S700). The second generation function 150b generates, from the generated second ultrasound data 3, a second ultrasound image 22 representing a single image (Step S800). The second ultrasound image 22 corresponds to a single image in which fluid (blood flow) in the first ultrasound data 10 representing the first number of images is locally extracted or enhanced.

The second generation function 150b also generates second ultrasound data 3 from first ultrasound data 10 representing a second number of images that is greater than the first number (Step S900). The second generation function 150b generates, from the generated second ultrasound data 3, a second ultrasound image 23 representing a single image (Step S1000). The second ultrasound image 23 corresponds to a single image in which fluid (blood flow) in the first ultrasound data 10 representing the second number of images is locally extracted or enhanced.

That is, the second generation function 150b generates two pieces of the second ultrasound data 3 (ultrasound data representing two images) by changing the number of pieces (the number of frames or analysis time) of the first ultrasound data 10. In this case, as the number of pieces of the first ultrasound data 10 increases, contrast or sharpness of an ultrasound image based on the second ultrasound data is improved but sensitivity may be reduced, and noise is reduced but responsiveness may be lowered. Specifically, as compared with the second ultrasound image 21 based on the second ultrasound data 3 generated from four frames of the first ultrasound data 10, the second ultrasound image 23 based on the second ultrasound data 3 generated from eight frames of the first ultrasound data 10 is improved in contrast and sharpness but may be reduced in sensitivity, and is reduced in noise but may be lowered in responsiveness.

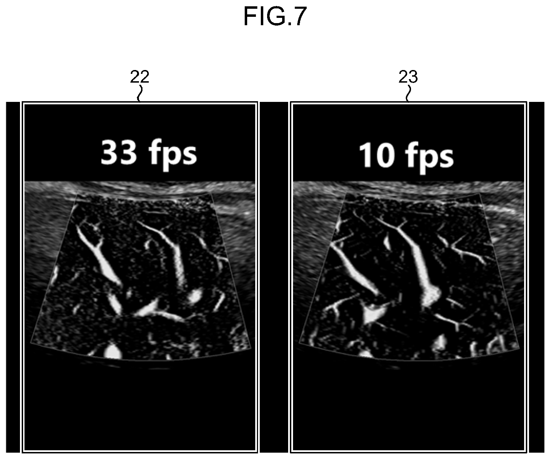

The display control function 150c displays a combination of the two second ultrasound images 22 and 23 in respective predetermined display regions of the display 135 (step S1100). That is, a plurality of different images for the same part of the subject are displayed on the display 135. As illustrated in FIG. 7, the display control function 150c displays the two second ultrasound images 22 and 23 side by side. The display control function 150c may switch and display the second ultrasound images 22 and 23 in a predetermined display region of the display 135.

As described above, according to the second embodiment, the two pieces of the second ultrasound data 3 (ultrasound data representing two images) are generated by changing the number of pieces (the number of frames or analysis time) of the first ultrasound data 10, and the two second ultrasound images 22 and 23 for the same part of the subject are displayed on the display 135 based on the generated second ultrasound data. Therefore, it is possible to reduce a problem of trade-off caused by processing for improving image quality of an ultrasound image.

In the present embodiment, the example in a case where the two second ultrasound images 22 and 23 are displayed on the display 135 has been described, but a plurality of second ultrasound images for the same part of the subject may be displayed on the display 135 individually. For example, the second generation function 150b may generate a plurality of pieces of the second ultrasound data 3 (a plurality of pieces of the frame data) by changing the number of pieces (the number of frames or analysis time) of the first ultrasound data 10, and generate a plurality of second ultrasound images based on the generated pieces of second ultrasound data. In this case, the display control function 150c may display a combination of the second ultrasound images on the display 135.

### Third Embodiment

In the first embodiment and the second embodiment, as illustrated in FIGS. 5 and 7, the example in a case where the images (for example, the first ultrasound image 20 and the second ultrasound image 21, or the two second ultrasound images 22 and 23) for the same part of the subject are displayed on the display 135 has been described. In the present embodiment, an example in a case where a plurality of images for different parts of the subject are displayed on the display 135 will be described.

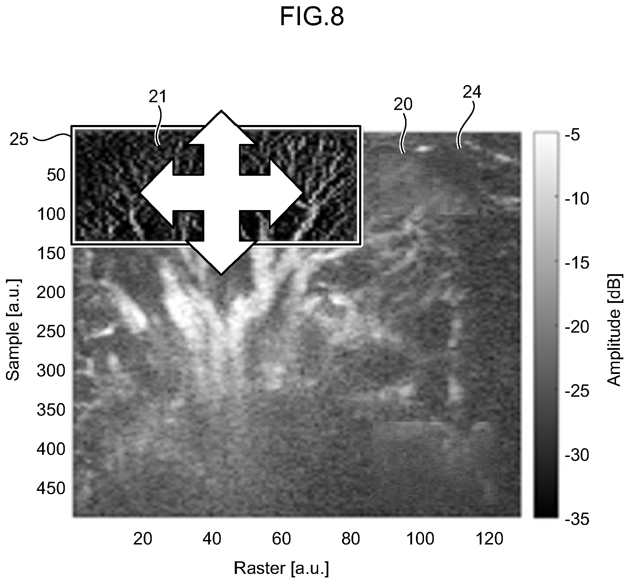

For example, as illustrated in FIG. 8, the display control function 150c may superimpose the first ultrasound image 20 and the second ultrasound image 21 within a predetermined display range of the display 135, display a portion of the first ultrasound image 20 corresponding to one region 24 of the display range on the display 135 in a cropped state in the one region 24, and display a portion of the second ultrasound image 21 corresponding to the other region 25 of the display range on the display 135 in a cropped state in the other region 25.

In other words, the display control function 150c may set the region 25 as a region of interest (ROI) in which the second ultrasound image 21 is displayed in a state where the first ultrasound image 20 is being displayed on the display 135. In this case, the display control function 150c may move the region 25 on the first ultrasound image 20 in response to a user operation via the input device 134, and display a portion of the second ultrasound image 21 corresponding to the moved region 25 on the display 135 in a cropped state in the moved region 25.

As described above, according to the third embodiment, the images (for example, the first ultrasound image 20 and the second ultrasound image 21) for different parts of the subject are displayed on the display 135. Therefore, while observing, in a partial region, the second ultrasound image 21 in which contrast or sharpness is improved and noise is reduced, it is possible to observe the first ultrasound image 20 that is displayed around the second ultrasound image 21 and has higher sensitivity or responsiveness than the second ultrasound image 21, and to reduce a problem of trade-off caused by processing for improving image quality of an ultrasound image.

As the other example, the display control function 150c may superimpose the two second ultrasound images 22 and 23 described in the second embodiment within a predetermined display range of the display 135, display a portion of one second ultrasound image 22 corresponding to one region of the display range in a cropped state in the one region, and display a portion of the other second ultrasound image 23 corresponding to the other region of the display range in a cropped state in the other region.

In other words, the display control function 150c may set an ROI in which the other second ultrasound image 23 is displayed in a state where one second ultrasound image 22 of two second ultrasound images 22 and 23 is being displayed on the display 135. In this case, the display control function 150c may move the ROI on the second ultrasound image 22 in response to a user operation via the input device 134, and display a portion of the second ultrasound image 23 corresponding to the moved ROI on the display 135 in a cropped state in the moved ROI.

In the present embodiment, the example in a case where two ultrasound images (for example, the first ultrasound image 20 and the second ultrasound image 21, or the two second ultrasound images 22 and 23) for different parts of the subject are displayed on the display 135 has been described, but a plurality of ultrasound images (for example, the first ultrasound image 20 and the second ultrasound images 21, 22, and 23) for different parts of the subject may be displayed on the display 135.

### Other Embodiments

In the first embodiment, the example in a case where data obtained by performing addition/time-averaging (persistence) on the pieces of the frame data 1a, 1b, and 1c for every fixed number of frames (FIG. 3 illustrates five frames) is used as the first ultrasound data 10 has been described, but a plurality of pieces of frame data 1a', 1b', and 1c' from which signal components representing local peaks of fluid are extracted may be generated as the first ultrasound data. In this case, the processing for improving image quality of the first ultrasound image 20 represented by the first ultrasound data may be processing other than the processing described in the first embodiment.

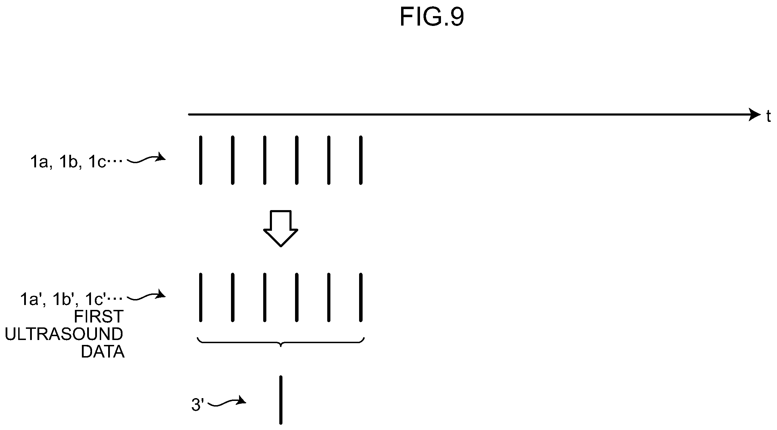

For example, as illustrated in FIG. 9, the first generation function 150a sequentially acquires a plurality of pieces of frame data 1a, 1b, 1c, ... that are sequentially collected based on transmission and reception of ultrasound waves by the ultrasound probe 105. The first generation function 150a extracts signal components representing local peaks of fluid (local peak signals) in individual frames from the pieces of frame data 1a, 1b, 1c, .... The signal components representing the local peaks of the fluid are signals having relatively high signal values (amplitudes) in a local range of a frame. For extraction of the local peak signals, the kernel 30 used in the first embodiment may be used, or other methods may be used.

For example, the local peak signals may be extracted as signal components representing local peaks of fluid (local peak signals) in individual frames by performing processing for sharpening local peaks on the pieces of frame data 1a, 1b, 1c, .... In this case, the first generation function 150a performs processing for increasing effective resolution by performing resampling on each of the pieces of frame data 1a, 1b, 1c, ... to increase pixel density. In the resampling, for example, each pixel may be replaced with a plurality of pixels having a smaller size. A signal value of each pixel after the replacement may be interpolated from an original signal value (a signal value before the replacement) by, for example, bicubic interpolation or the like. The first generation function 150a may sharpen the local peak signals by raising pixel values, represented by the individual pieces of frame data 1a, 1b, 1c, ... after the effective resolution is increased, to a power (for example, an 8th power, a 12th power, or the like). That is, by raising the pixel values to a power, a pixel having a pixel value higher than that of its surroundings is extracted as a pixel representing a local peak of fluid. The first generation function 150a may extract local peak signals for individual frames by performing threshold processing or the like on the pieces of frame data 1a, 1b, 1c, ... after pixel values are sharpened. The first generation function 150a performs extraction processing of local peak signals on pieces of frame data sequentially acquired as first ultrasound data, and generates a plurality of pieces of frame data 1a', 1b', 1c', ... from which the local peak signals are extracted, as the first ultrasound data.

Then, the second generation function 150b generates second ultrasound data 3 representing a single image in which fluid is locally extracted or enhanced, by synthesizing the pieces of frame data 1a', 1b', 1c', ... for every fixed number of frames (FIG. 9 illustrates six frames). That is, the second generation function 150b may generate second ultrasound data 3' representing a single image based on the first ultrasound data (pieces of frame data 1a', 1b', 1c', ...) representing a plurality of images continuous in a time direction, and generate a second ultrasound image (local peak sharpened image) based on the second ultrasound data 3'. Then, even in a case where such processing is performed, there is the problem of trade-off described in the above embodiment. That is, in the second ultrasound image 21 based on the second ultrasound data 3', the fluid represented by the first ultrasound data 10 is locally extracted or enhanced, so that contrast or sharpness is improved as compared with the first ultrasound image 20 but sensitivity may be reduced, and noise is reduced as compared with the first ultrasound image 20 but responsiveness may be lowered. As illustrated in FIG. 5, the display control function 150c displays a combination of the first ultrasound image 20 and the second ultrasound image 21 based on the first ultrasound data (pieces of frame data 1a', 1b', 1c', ...) in a predetermined display region of the display 135.

As described above, even in a case where different processing for improving image quality of the first ultrasound image 20 represented by the first ultrasound data is employed, the second ultrasound image 21 in which contrast or sharpness is improved and noise is reduced is displayed on the display 135 together with the first ultrasound image 20 having higher sensitivity or responsiveness than the second ultrasound image 21. Therefore, the problem of trade-off caused by the processing for improving image quality of the ultrasound image can be reduced. The above-described processing is applicable also in a case where a plurality of the second ultrasound images (second ultrasound images 22 and 23) are displayed on the display 135 for the same part of the subject. For example, the second generation function 150b may generate a plurality of pieces of the second ultrasound data 3 (a plurality of pieces of the frame data) by changing the number of pieces (the number of frames or analysis time) of the first ultrasound data (the pieces of frame data 1a', 1b', 1c', ...), and generate and display a plurality of second ultrasound images based on the pieces of the second ultrasound data 3.

As the other embodiment, the display control function 150c may use, for at least one image among a plurality of ultrasound images (for example, the first ultrasound image 20 and the second ultrasound images 21, 22, 23) to be displayed on the display 135, an image representing a result of performing analysis processing or image processing. For example, the display control function 150c may perform processing for generating an image representing directions or magnitudes of blood flow with vector symbols on the second ultrasound data 3, which is a source of the second ultrasound image 22 illustrated in FIG. 5, and display an image representing a result of the processing as the second ultrasound image on the display 135. The display control function 150c may also perform tracking processing of blood flow speckles on the second ultrasound data 3, which is a source of the second ultrasound image 23 illustrated in FIG. 7, and display an image representing a result of the tracking processing as the second ultrasound image on the display 135.

The second generation function 150b may generate second ultrasound data representing a single image by performing peak hold processing on first ultrasound data representing a plurality of images within a predetermined time period. In this case, the second generation function 150b may perform processing of extracting a local peak signal value in each of a plurality of images (frames) represented by the first ultrasound data, and holding a signal having the largest local peak signal at positions corresponding to each other between the images. The display control function 150c may display a second ultrasound image (peak hold image) based on the second ultrasound data obtained by the peak hold processing on the display 135 together with another ultrasound image.

The second generation function 150b may generate second ultrasound data representing a single image by performing the peak hold processing on the first ultrasound data representing the images within a predetermined time period. In this case, the second generation function 150b may perform processing of extracting a local peak signal value in each of a plurality of images (frames) represented by the first ultrasound data, and holding a signal having the largest local peak signal at positions corresponding to each other between the images. The display control function 150c may display a second ultrasound image based on the second ultrasound data obtained by the peak hold processing on the display 135 together with another ultrasound image. The second generation function 150b may generate second ultrasound data representing a single image by performing peak hold processing on first ultrasound data representing a plurality of images within a predetermined time period. In this case, the second generation function 150b may perform processing of extracting a local peak signal value in each of a plurality of images (frames) represented by the first ultrasound data, and holding a signal having the largest local peak signal at positions corresponding to each other between the images.

Furthermore, the display control function 150c may adaptively change the image to be displayed on the display 135. For example, the first ultrasound image 20 and the second ultrasound image 21 illustrated in FIG. 5, the second ultrasound images 22 and 23 illustrated in FIG. 7, or the like may be adaptively selected and displayed. The display control function 150c may select a plurality of images to be displayed on the display 135 based on an eigenvalue during application of a wall filter when generating a fluid signal. For example, in a case where a difference between a first eigenvalue and a second eigenvalue during application of a wall filter when generating a fluid signal is large, the display control function 150c may determine that there is little movement, adaptively select the first ultrasound image (blood flow image) and the peak hold image (second ultrasound image) as images to be displayed on the display 135, and display these images. In a case where the difference between the first eigenvalue and the second eigenvalue during application of a wall filter when generating a fluid signal is small, the display control function 150c may determine that movement is large, select the first ultrasound image (blood flow image) and the local peak sharpened image (second ultrasound image) as images to be displayed on the display 135, and display these images.

In the above-described embodiments, the blood flow (blood) is described as an example of the fluid moving inside the body of the subject, but the fluid may be a contrast agent.

Each of the components of each of the apparatuses illustrated in the description of the above-described embodiments is a functional concept, and does not necessarily have the physical configuration as illustrated in the figures. That is, the specific form of distribution and integration of each of the apparatuses is not limited to those illustrated in the figures, and all or some thereof can be functionally or physically distributed and integrated in any units according to various loads and usage conditions. Furthermore, each of the processing functions performed by each of the apparatuses can be implemented, in all or certain parts thereof, by CPU and a computer program analyzed and executed by the CPU, or by hardware using wired logic.

The method described in the above-described embodiments can be implemented by executing a computer program prepared in advance on a computer such as a personal computer or a workstation. This computer program can be distributed via a network such as the Internet. This computer program is recorded on a non-transitory computer readable recording medium such as a hard disk, a flexible disk (FD), a CD-ROM, an MO, a DVD, a flash memory such as a USB memory and an SD card memory, or the like, and can also be executed by a computer reading the computer program from the non-transitory computer readable recording medium.

As described above, according to the embodiments, it is possible to stably provide an image having high visibility.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims.

## Claims

1. A medical information processing apparatus comprising a processing circuit (150) configured to:
generate first ultrasound data (10) representing a fluid inside a body of a subject based on a received signal of an ultrasound wave received by an ultrasound probe (105);
generate second ultrasound data (3) by performing processing of locally extracting or enhancing the fluid represented by the first ultrasound data (10); and
display a combination of a first ultrasound image based on the first ultrasound data (10) and a second ultrasound image based on the second ultrasound data (3), or a combination of a plurality of the second ultrasound images based on a plurality of pieces of the second ultrasound data (3) on a display (135).

2. The medical information processing apparatus (110) according to claim 1, wherein
the processing circuit (150) is configured to:
generate the second ultrasound data (3) representing a single image in which the fluid is locally extracted or enhanced, from the first ultrasound data (10) and representing a plurality of images within a predetermined time period; and
display the combination of the first ultrasound image based on the first ultrasound data (10) and the second ultrasound image based on the second ultrasound data (3) on the display (135).

3. The medical information processing apparatus (110) according to claim 2, wherein
the processing circuit (150) is configured to:
superimpose the first ultrasound image and the second ultrasound image within a predetermined display range of the display (135);
display a portion of the first ultrasound image corresponding to one region of the display range in a cropped state in the one region; and
display a portion of the second ultrasound image corresponding to an other region of the display range in a cropped state in the other region.

4. The medical information processing apparatus (110) according to either of claims 1 or 2, wherein
the processing circuit (150) is configured to display the first ultrasound image and the second ultrasound image side by side on the display (135).

5. The medical information processing apparatus (110) according to either of claims 1 or 2, wherein
the processing circuit (150) is configured to display the first ultrasound image and the second ultrasound image on the display (135) in a switched manner.

6. The medical information processing apparatus (110) according to claim 1, wherein
the processing circuit (150) is configured to:
generate two pieces of the second ultrasound data (3), each representing a single image in which the fluid is locally extracted or enhanced, from the first ultrasound data (10) representing a first number of images and the first ultrasound data (10) representing a second number of images greater than the first number; and
display a combination of two second ultrasound images based on the two pieces of the second ultrasound data (3) on the display (135).

7. The medical information processing apparatus (110) according to claim 6, wherein
the processing circuit (150) is configured to:
superimpose the two second ultrasound images within a predetermined display range of the display (135);
display a portion of one of the second ultrasound images corresponding to one region of the display range in a cropped state in the one region; and
display a portion of an other of the second ultrasound images corresponding to an other region of the display range in a cropped state in the other region.

8. The medical information processing apparatus (110) according to claim 6, wherein
the processing circuit (150) is configured to display the two second ultrasound images side by side on the display (135).

9. The medical information processing apparatus (110) according to claim 6, wherein
the processing circuit (150) is configured to display the two second ultrasound images on the display (135) in a switched manner.

10. An ultrasound diagnosis apparatus comprising:
the medical information processing apparatus of any of claims 1 to 9; and
an ultrasound probe (105).

11. A computer implemented method of processing signals from an ultrasound probe, the method comprising:
generating first ultrasound data (10) representing a fluid inside a body of a subject based on a received signal of an ultrasound wave received by an ultrasound probe (105);
generating second ultrasound data (3) by performing processing of locally extracting or enhancing the fluid represented by the first ultrasound data (10); and
displaying, on a display (135), a combination of a first ultrasound image based on the first ultrasound data (10) and a second ultrasound image based on the second ultrasound data (3), or a combination of a plurality of the second ultrasound images based on a plurality of pieces of the second ultrasound data (3).

12. A program that causes a computer to execute:
generating first ultrasound data (10) representing a fluid inside a body of a subject based on a received signal of an ultrasound wave received by an ultrasound probe (105);
generating second ultrasound data (3) by performing processing of locally extracting or enhancing the fluid represented by the first ultrasound data (10); and
displaying, on a display (135), a combination of a first ultrasound image based on the first ultrasound data (10) and a second ultrasound image based on the second ultrasound data (3), or a combination of a plurality of the second ultrasound images based on a plurality of pieces of the second ultrasound data (3).
